# EUROPEAN PATENT SPECIFICATION

(11) **EP 3 334 533 B1**
(45) Date of publication and mention of the grant of the patent: **21.04.2021**
(21) Application number: 16714508.5
(22) Date of filing: 04.03.2016
(51) Int. Cl.: C12Q 1/6844, C12Q 1/6851, C12Q 1/686, B01L 7/00

(54) **MULTIPLEXED DETECTION OF NUCLEIC ACID TARGETS DIRECTLY FROM SAMPLES CONTAINING BLOOD**
MULTIPLEXIERTER NACHWEIS VON NUKLEINSÄURETARGETS DIREKT AUS BLUTHALTIGEN PROBEN
DÉTECTION MULTIPLEXÉE DE CIBLES D'ACIDES NUCLÉIQUES DIRECTEMENT À PARTIR D'ÉCHANTILLONS CONTENANT DU SANG

(30) Priority: 05.03.2015 GB 201503775
(43) Date of publication of application: 20.06.2018
(73) Proprietor: BG Research Ltd, Cambridgeshire PE28 0NJ (GB)
(72) Inventor: NAZARETH, Nelson, Upper Dean Cambridgeshire PE28 0ND (GB); EDGE, David, Warlingham Surrey CR6 9RP (GB); TYLER, Adam, Kettering Northamptonshire NN15 5TS (GB)
(74) Representative: Potter Clarkson
(86) International application number: PCT/GB2016/000046
(87) International publication number: WO 2016/139443

(56) References cited:
- "MxPro QPCR Software for Mx3000P and Mx3005P QPCR Systems", , 1 March 2009 (2009-03-01), pages 1-433, XP55276973, Retrieved from the Internet: URL:http://www.agilent.com/cs/library/user manuals/public/MxPro_Manual.pdf [retrieved on 2016-06-01]
- ZHIAN ZHANG ET AL: "Direct DNA Amplification from Crude Clinical Samples Using a PCR Enhancer Cocktail and Novel Mutants of Taq", THE JOURNAL OF MOLECULAR DIAGNOSTICS, vol. 12, no. 2, 1 March 2010 (2010-03-01), pages 152-161, XP055001258, ISSN: 1525-1578, DOI: 10.2353/jmoldx.2010.090070
- A. CASTLEY: "Clinical Applications of Whole-Blood PCR with Real-Time Instrumentation", CLINICAL CHEMISTRY, vol. 51, no. 11, 1 November 2005 (2005-11-01), pages 2025-2030, XP055167243, ISSN: 0009-9147, DOI: 10.1373/clinchem.2005.055327
- BRIAN J TAYLOR ET AL: "Real-time PCR detection of Plasmodium directly from whole blood and filter paper samples", MALARIA JOURNAL, BIOMED CENTRAL, LONDON, GB, vol. 10, no. 1, 19 August 2011 (2011-08-19), page 244, XP021092124, ISSN: 1475-2875, DOI: 10.1186/1475-2875-10-244
- LUTZ ERIC LEHMANN ET AL: "A multiplex real-time PCR assay for rapid detection and differentiation of 25 bacterial and fungal pathogens from whole blood samples", MEDICAL MICROBIOLOGY AND IMMUNOLOGY, SPRINGER, BERLIN, DE, vol. 197, no. 3, 16 November 2007 (2007-11-16), pages 313-324, XP019630543, ISSN: 1432-1831
- Evaldas Katilius ET AL: "Multiphoton excitation of fluorescent DNA base analogs", Journal of biomedical Optics, vol. 11, no. 4, 1 January 2006 (2006-01-01), page 044004, XP055655479, ISSN: 1083-3668, DOI: 10.1117/1.2337521

## Description

### Field of Invention

The present invention relates to a process and apparatus for the multiplexed detection of nucleic acid targets directly from samples containing blood. A pertinent example is the concurrent detection of a target virus together with a human control gene amplified from the human DNA present in the white blood cells.

### Background

The limited number of publications in this area suggest that the commonly used fluorescently labelled real-time PCR methodology cannot be utilised in the presence of any significant amounts of blood in the sample to be tested. It would clearly be advantageous for researchers and clinicians to be able to rapidly detect both pathogens and human targets directly from a blood sample, for example at the point of care, without the need to perform time consuming nucleic acid extraction.

The background to this invention lies in two distinct fields. The first is the applicant's own background knowledge in the fields of ultra-rapid PCR and the extension of this to the ability to directly amplify nucleic acids from a crude sample in a single closed tube format. The requirement for diagnostic tests that are both sensitive and take an absolute minimum time to detection has been clearly established, for example in the face of an emerging disease outbreak where in field screening would save many lives. The applicants have previously demonstrated direct rapid detection of target species from a number of matrices. One target which is both desirable and technically challenging is the direct screening of blood borne pathogens direct from a whole blood sample. The challenges comprise, firstly the amount of blood that can be accepted into the reaction and secondly the impact of the presence of this blood on the assay outcome.

The second relevant area of background information is the work of the group Barnes *et al* (Mutants of Taq DNA polymerase resistant to PCR inhibitors allow DNA amplification from whole blood and crude soil samples) (full reference of any publication is needed). There are also evolved enzymes and adjuncts designed to make direct PCR in the presence of inhibitors a possibility. This work showed that it is possible through mutation of the polymerase to make enzymes capable of amplifying DNA in the presence of whole blood. The same paper highlights the issue that the present invention seeks to overcome, fluorescence inhibition observed by the presence of human blood.

When real-time PCR is completed in the presence of whole human blood in a standard real-time thermal cycler there is observed a very high background and additionally an attenuation of fluorescence signals in the order of 85-95% depending on the wavelength of the dyes involved. The inventor has sought to overcome this with an intercalating dye approach but the significantly high background level from the whole human genomic DNA in the blood sample renders this approach both unsuited to clinical use but also makes multiplexing impossible. All clinical assays will have some form of internal control nucleic acid species and as such it has not been possible to perform direct diagnostic detection of target species from a whole blood sample.

The inventor has repeated earlier experiments to determine the effects of blood on fluorescence collection, however there is little background literature relating to the field of PCR since it was assumed it would not work. However, there are some papers in the field of directly fluorescently imaging blood for examining the brain and other organs. The data effectively shows why there is an 85-95% reduction in fluorescence - rendering direct multiplexed detection of nucleic acid targets direct from whole blood impossible. Firstly, there are strong absorption peaks at around 541 and 577nm and these completely absorb the fluorescence output from any common dyes in these wavelengths. Secondly, there is an observable red-shift; standard systems have narrow 10-20nm band pass filters in front of the detectors and any wavelength shift will clearly move a proportion of the light away from the wavelengths which will be collected. Thirdly, there is a strength quenching effect from the presence of the haemoglobin itself. In summary the commonly used combination of wavelength insensitive detector and the use of filters to enable the direct multiplexed detection of multiple nucleic acid targets in a single closed tube format will not work in the presence of blood.

The present invention comprises therefore a method for the detection of one or more nucleic acid targets from a sample containing whole blood.

Mx Pro QPCR Software for Mx3000P and Mx3005P QPCR Systems, XP 055276973 is the manual of a PCR machine Stratagene MX-PRO, and discusses the use of red dyes in the context of real-time PCR labels.

Castley 2005 Clinical Chemistry 51: 2025-2030 discusses a method of performing genotyping on "whole-blood" using a real-time PCR instrument.

### Summary of the Invention

The inventors discovered that beyond the large quenching effect up to 620nm there is a much lower effect of the presence of blood, in the region of 20-30% as opposed to 85-95% inhibition and as such the system provides a means of delivering high excitation powers of red light to the sample. The second key feature is the ability to detect the emitted fluorescence signals by the use of a spectrophotometer calibrated to collect light at 650nm and ranging as far as 750nm. By use of high excitation power red light and a spectrophotometer it becomes possible, via means of a spectral deconvolution approach, to simultaneously detect multiple target species in a single fluorescence reading. A pertinent example may be to distinguish between an infection resulting from the filovirus family while excluding infections from organisms causing similar symptoms and still have spectral space for an internal control. Standard PCR optical approaches use single point excitation sources in combination with emission filters and as such there would neither be sufficient separation between dye emission wavelengths or the ability to remove cross-talk between these channels. Systems do exist that have a red or far red excitation component, but these do not allow high level multiplexing when there is an absolute requirement for.

The present invention is defined by the claims and provides:
a method for the multiplexed detection of nucleic acid targets directly amplified in a reaction with a sample when the sample contains blood, the method characterized by excitation of the reaction at a wavelength greater than 620nm and wherein the excitation is effected with a power of at least 2mW.

The invention also provides:
the use of an apparatus for the multiplexed detection of nucleic acid targets according to the method of any one of claims 1-7 wherein the apparatus is characterized by a reaction chamber (17) arranged for effecting the amplification and means (18) for the delivery to the sample of red excitation light, at greater than 620nm wavelength, and having excitation means (18) arranged to operate with a power of at least 2mW.

In one embodiment, the method is a method for the multiplexed detection of multiple nucleic acid targets directly amplified in a single closed tube format when the sample includes whole blood and comprises the delivery to the sample of red excitation light, at greater than 620nm wavelength, at a power in the order of at least 2mW but preferably in excess of 5mW. In a preferred embodiment this excitation is delivered by a laser diode at 635nm but a range of 633-642 has proven to be applicable. To remove light emitting from the laser below the 640nm wavelength range either a band pass filter or short pass may be used. In an alternative embodiment LED excitation has been provided via coupling the LED into a large diameter core, high NA fiber and is similarly band pass filtered. Additionally a free space approach with regard to excitation has been demonstrated but the preferred embodiment is the fiber based delivery approach.

Also disclosed is an apparatus for the multiplexed detection of multiple nucleic acid targets directly amplified when the sample includes whole blood, the apparatus comprising a reaction chamber arranged for effecting the amplification and means for the delivery to the sample of red excitation light, at greater than 620nm wavelength.

The excitation light is preferably delivered at a power in the order of at least 2mW but even more preferably in excess of 5mW. The excitation may be delivered by a laser diode at 635nm but a range of 633-642 has proven to be applicable. To remove light emitting from the laser below the 640nm wavelength range either a band pass filter or short pass may be used. In an alternative disclosure LED excitation has been provided via coupling the LED into a large diameter core, high NA fiber and is similarly band pass filtered. Additionally a free space approach with regard to excitation has been demonstrated but the preferred disclosure is the fiber based delivery approach. The apparatus may incorporate a spectrophotometer for collation of the resultant emission profile.

In the preferred disclosure the system takes the form of an eight well randomly accessed system for direct in field detection (GB2015000027). This previous specification describes a system that utilises the HRM approach (US8597937) to enable direct freeze/thaw (EP2585581) mediated lysis and combined amplification in a single closed tube format. The eight well format means that all of the eight reaction vessels must transmit fluorescence signals to a single shared spectrophotometer and as such an optical fiber array forms the preferred disclosure. This consists of an octofurcated fiber array consisting of eight 200 micron core 0.22NA fibers each terminated to eight individual laser diode modules. These comprising a laser diode, control electronics, an aspheric lens for collimation and a band or short pass filter such that only light below 640nm is emitted. Each of these laser diodes is temperature controlled via means of being included within an off shoot of the HRM mechanism (US8597937) such that their temperature is held constant; laser diodes shift wavelength and excitation power with temperature shift and as such much be stabilised. The octofurcated delivery fibers are conjoined at a junction with eight collection fibers such that a double core fiber is terminated over the top of each sample reaction vessel, with one providing the excitation and a second collecting the emitted fluorescence. The eight collection fibers are brought together at the SMA connector terminus into an array of two by four as per the attached drawings and these are focused onto the spectrophotometer after passing through a long-pass filter that rejects any light below 650nm, such that only the intended emission light is imaged onto the spectrophotometer.

In use the preferred apparatus operates in a sequential fashion such that the illumination of each individual vessel in turn images one spectrum onto the detector. A means of electronic control then synchronises this illumination with both the time base and the heating system such that the contemporaneous reading from each well is taken at the correct time point.

It can clearly be envisaged that disclosures from one vessel with free space illumination to a single spectrophotometer, through different numbers of spectrophotometers and indeed differing fiber arrays can be constructed that meet the central tenet - the system must have sufficient excitation power at a wavelength greater than 620nm and preferably utilise a spectrophotometer for collation of the resultant emission profile.

### Brief Description of the Drawings

Embodiments of the invention will now be particularly described by way of example with reference to the accompanying drawings of which:
Figure 1a is a graph of relative detected intensity against excitation light wavelength, for various integration times with a no fluorophore negative control sample and no blood present;
Figure 1b is a graph as in figure 1a but with an intercalating dye and a DNA present;
Figure 1c is a graph as in figure 1a but when blood is present;
Figure 1d is a graph as in figure 1b but when blood is present;
Figure 2 is a graph showing the fluorescence arising from the use of red excitation;
Figure 3 is a perspective view of, an eight well field PCR apparatus;
Figure 4 is a plan view of the apparatus depicted in figure 3
Figure 5 is a cross section of the head array of the apparatus of figure 3;
Figure 6 is a plan view of the optical arrangement in the apparatus of figure 3;
Figure 7 depicts an optical excitation source array for the apparatus of figure 3;
Figure 8 shows a spectrophotometer;
Figure 9 shows an optical fibre array 13; and

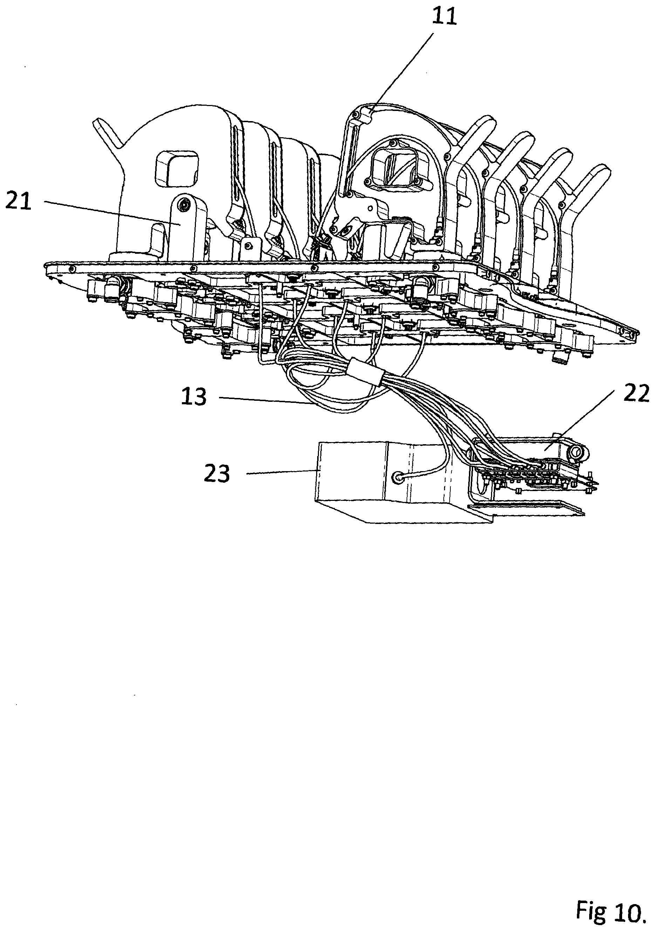

Figure 10 shows a complete PCR apparatus exposed to show the PCR system and the optics.

### Description of the Preferred Disclosure

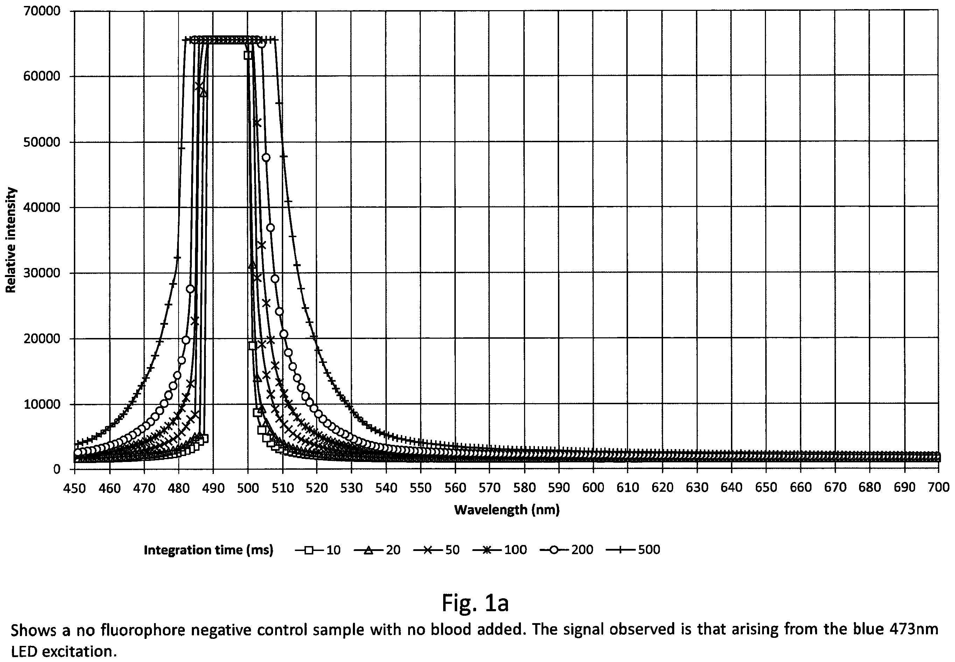

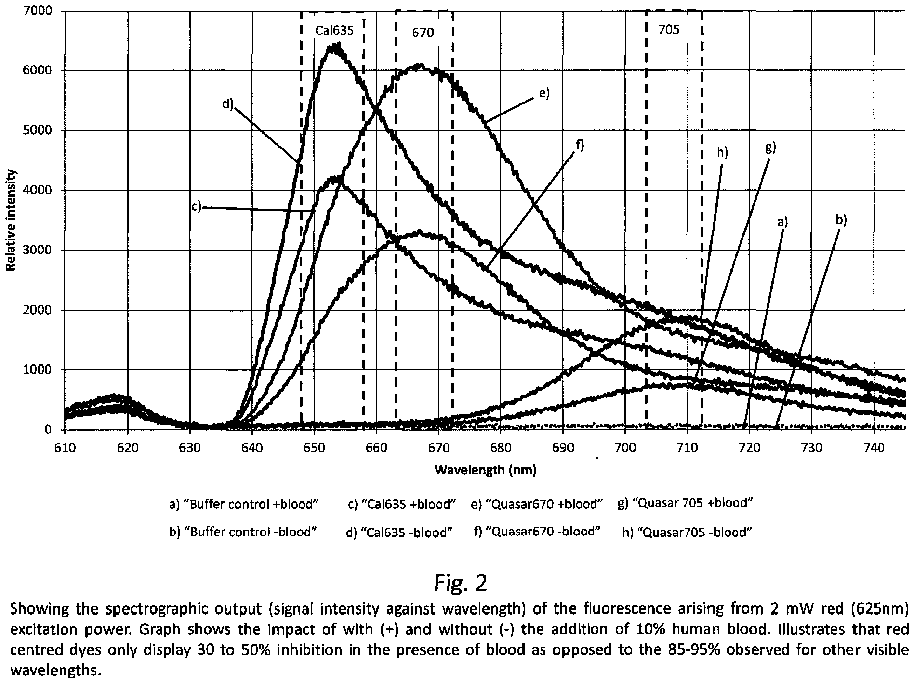

Figures 1a-d and 2 show the effect of blood on the signal emanating from fluorescence in a sample undergoing PCR, with the vertical axes showing the relative intensity of the light and the horizontal axis showing the wavelength in nanometers (nm).

Figure 1a depicts the results from a control sample, that is one which contains no blood. The signal observed is that arising from a blue 473nm LED excitation.

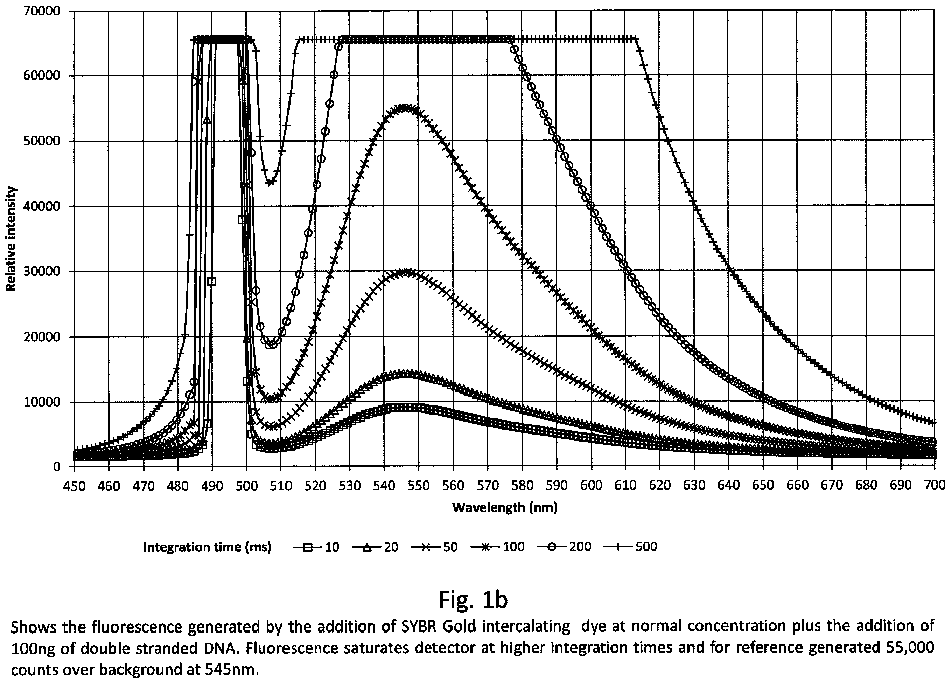

Figure 1b shows the fluorescence generated by the addition of SYBR gold intercalating dye at normal concentration including the addition of 100ng (nanograms) of double stranded DNA. It shows the fluorescence saturating the detector at the higher integration times. For reference, 55,000 counts were generated over the background at 545nm.

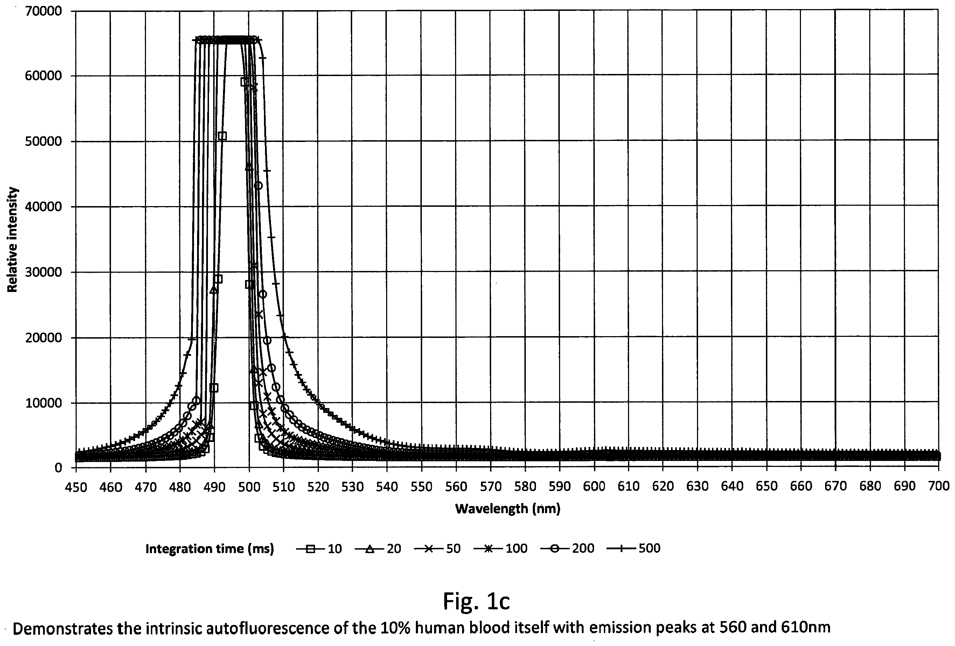

Figure 1c shows the effect of adding 10% by volume human blood to the sample. There is an intrinsic autofluorescence with emission peaks at 560 and 610nm.

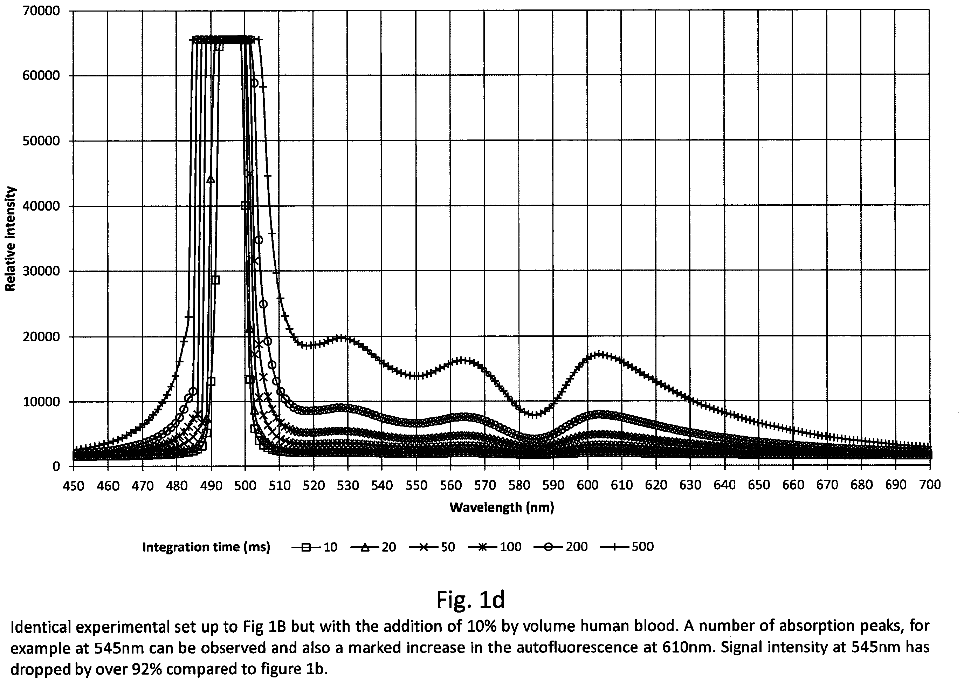

Figure 1d shows the results of setting up an identical experiment to that of figure 1b but with the addition of 10% by volume human blood to the sample. A number of absorption peaks, for example at 545nm can be observed, and also a marked increase in the autofluorescence at 610nm. Signal intensity at 545nm has dropped by over 92% compared to figure 1 b.

The spectrographic output of the fluorescence from samples at 2mW red (625nm) under different parameters is shown in figure 2. The graphs show the impact of with (+) and without (-) the addition of 10% by volume human blood. It illustrates that red centred dyes Cal635, Quasar70 and Quasar 705 only display 30 to 50% inhibition in the presence of blood as opposed to the 85-95% observed for other visible wavelengths.

The figures 1a-d and 2 demonstrate in summary that the present invention overcomes the greater than 90% inhibition of fluorescence signal observed using conventional qPCR optical approaches.

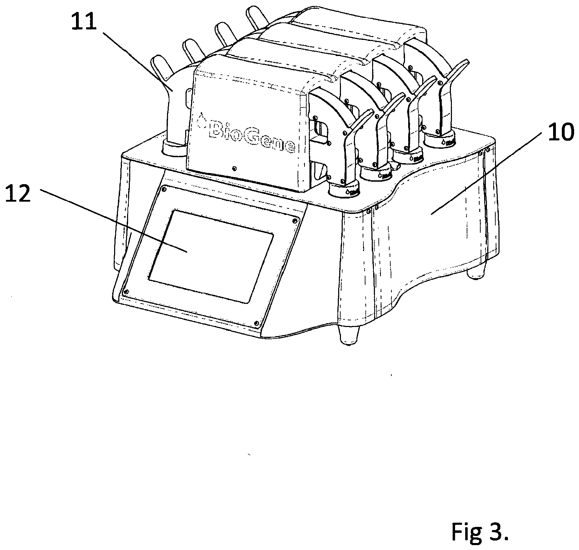

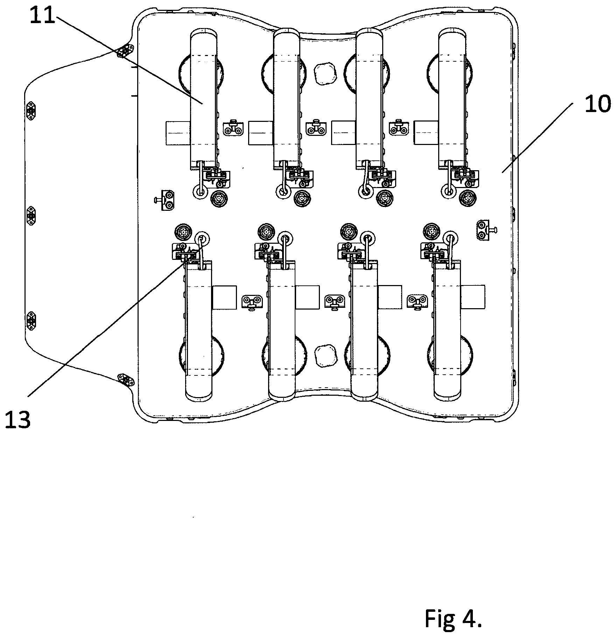

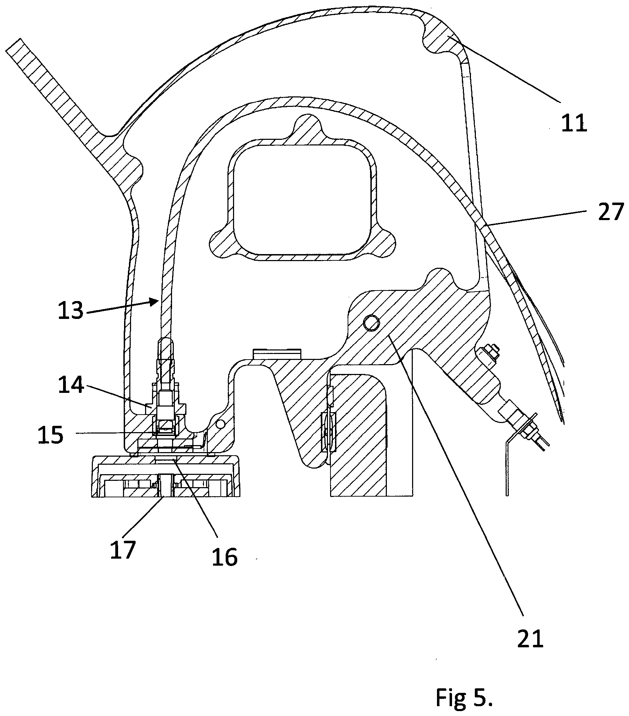

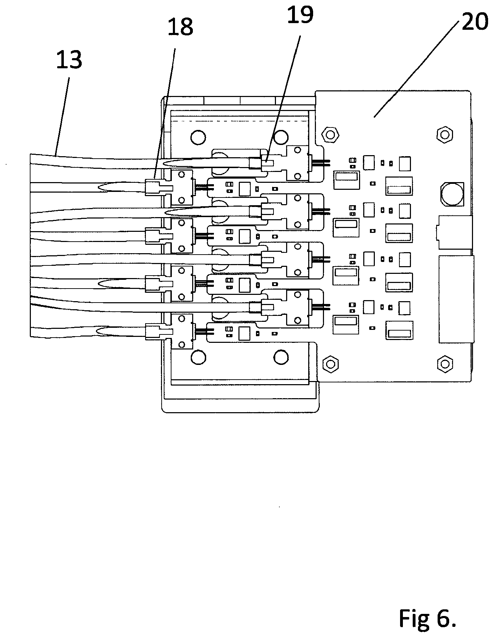

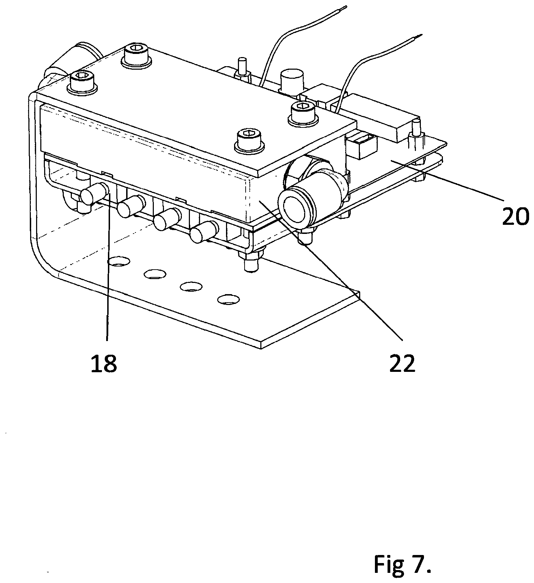

As shown in figures 3 to 9 a preferred disclosure is an instrument 10 for the field detection of amplified of nucleic acid targets in the presence of whole blood. The blood may be human or that of another creature - so long as it's red!

The instrument 10 comprises eight randomly accessible reaction stations each possessing an individual optical detection head 11. There is a display monitor 12 for presenting analysis results to the end user. Each optical head 11 contains one leg of an octofurcated fiber array 13 secured in place via an SMA connector 14. Within each of these connectors is located a collimating lens 15 such that the excitation beam and the emission light emanating from the reaction vessel are collected into the fiber 13. The excitation beam is focused through a clear lid 16 to a reaction vessel 17 and to a point at the base of the reaction vessel. The distal end of the fiber array 13 is attached to eight red laser diodes 18 contained in SMA housings and each with a bandpass filter 19 to prevent unwanted wavelengths being injected into the fiber. The laser diodes 18 are driven by a PCB 2 driving the current to each device such that the excitation power can be normalised across the array 13.

Each optical detection head 11 is mounted to the apparatus via a pivot 21 enabling the head 11 to be swivelled clear of the reaction vessel 16 so that the vessel 16 can be emplaced and removed from the heating/cooling apparatus whereby PCR can be performed on the content of the vessel.

A water cooling block 22 is arranged for stabilising the temperature of the laser diode 17 assembly.

In this disclosure a laser diode 18 emitting at 635-638nm is paired with a band pass filter in the laser housing that passes 630-642 but blocks other wavelengths at OD6.to excite fluorescence in the reaction vessel.

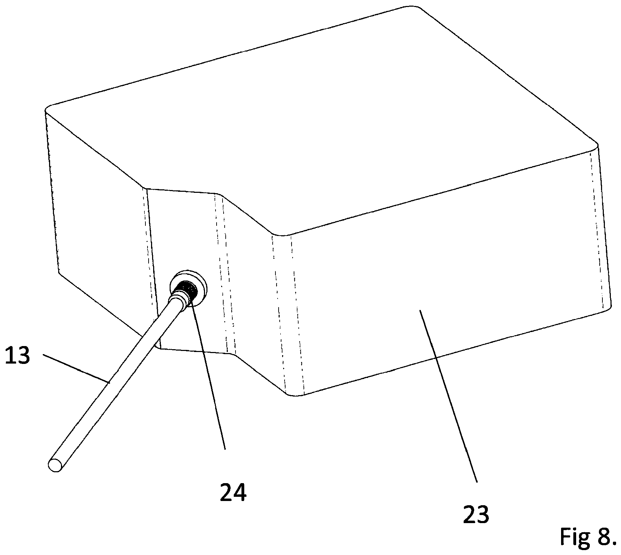

A spectrophotometer 23 (figure 8) is arranged to receive the light emission from the reaction vessels 17 via the fibre array 13 and a paired 650 or 665nm long-pass filter 24 at the entrance to the spectrophotometer 23.

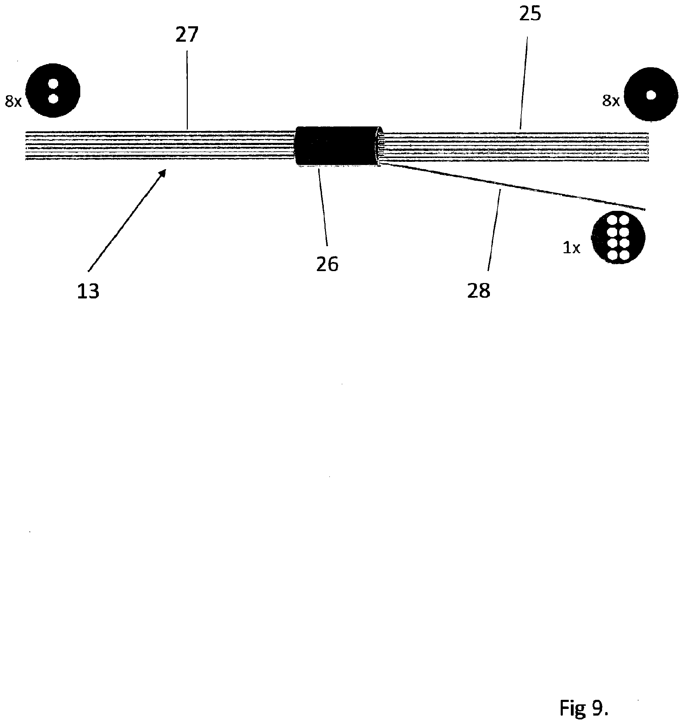

Figure 9 shows the optical fibre array 13. The fibre bundle 25 on the right comprises fibres attached to the laser diodes and contain a single core. These fibres enter a splitter 26 wherefrom emerge eight fibres now containing two cores 27, one leading back to the laser diodes 18 for excitation and the other leading to the spectrophotometer 23 for detection. The fibre 27 is the one located in the instrument pivoting head.. The leg leading to the spectrophotometer 23 exits the splitter having now been combined into a single fibre 28 containing eight cores in a two by four array

Not shown in the drawings is the means by which a PCR reaction is carried out in the reaction vessels 17. The reaction vessel 17, which is of microtitre capacity, is snugly held in a reaction vessel receiving cup which is associated with the working face of a peltier cell in such a way that a prescribed temperature can rapidly be reach in the reaction chamber of the reaction vessel. The base face of the peltier cell is associated with a heat source sink in which water is arranged to flow at a constant temperature intermediate the upper and lower temperatures of a PCR cycle.

In use whole human blood suspected of containing a potential target, such as a pathogen, is placed into the reaction vessel 17 with the correct primers and probes-labelled with for example Cy5.5, quasar 670, quasar 705 or any other multiplexed dyes known in the art. The laser is shone into the reaction vessel 17 as PCR proceeds and successful amplification generates spectra that are observed even in the presence of blood.

## Claims

1. A method for the multiplexed detection of nucleic acid targets directly amplified in a reaction with a sample when the sample contains blood, the method **characterized by** excitation of the reaction at a wavelength greater than 620nm and wherein the excitation is effected with a power of at least 2mW.

2. A method as claimed in claim 1 and carried out in a single closed tube format.

3. A method as claimed in claim 1 or claim 2 and wherein the amplification reaction comprises PCR, isothermal amplification, RT-PCR.

4. A method as claimed in any one of claims 1 to 3 and wherein subsequent processing comprises spectrally deconvoluting the multiple observed spectra arising from the plurality of signals being detected.

5. A method as claimed in any one of the preceding claims and wherein the excitation is delivered by laser or a light emitting diode (LED).

6. A method as claimed in any one of the preceding claims and comprising effecting and controlling the reaction employing a peltier cell having a working face arranged to heat and cool the reaction and a base face kept at a constant temperature intermediate the upper and lower temperatures of the reaction.

7. A method as claimed in any one of the preceding claims wherein the emitted fluorescence signals are detected by the use of a spectrophotometer, optionally calibrated to collect light at 650nm ranging as far as 750nm.

8. Use of an apparatus for the multiplexed detection of nucleic acid targets according to the method of any one of claims 1-7 wherein the apparatus is **characterized by** a reaction chamber (17) arranged for effecting the amplification and means (18) for the delivery to the sample of red excitation light, at greater than 620nm wavelength, and having excitation means (18) arranged to operate with a power of at least 2mW.

9. The use according to claim 8 wherein the apparatus has a fluorescence collection means comprising a spectrophotometer (23).

10. The use according to any one of claims 8 or 9 wherein the apparatus is arranged to deliver excitation at a range of 633-640nm.

11. The use according to any of claims 8-10 wherein the apparatus comprises excitation means (18) comprising one of a laser diode and a light emitting diode (LED).

12. The use according to any of claims 8-11 wherein the apparatus comprises a peltier cell having a working face arranged to heat and cool the reaction and a base face arranged to be kept at a constant temperature intermediate the upper and lower temperatures of the reaction.

13. The use according to any of claims 8-12 wherein the apparatus comprises a fibre array (13) arranged to convey light between the light source (18) and the reaction vessel and between the reaction vessel and a light detector.

14. The use according to any of claims 8-13 wherein the apparatus comprises an array of two by four collection fibers (13) that are focused onto a spectrophotometer (23) after passing through a 665nm longpass filter (24) such that only the intended emission light is imaged onto the spectrophotometer (23).

## Patentansprüche

1. Verfahren für den multiplexierten Nachweis von Nukleinsäurezielen, die in einer Reaktion mit einer Probe direkt amplifiziert werden, wenn die Probe Blut enthält, wobei das Verfahren durch eine Anregung der Reaktion bei einer Wellenlänge von über 620 nm gekennzeichnet ist und wobei die Anregung mit einer Leistung von wenigstens 2 mW ausgeführt wird.

2. Verfahren nach Anspruch 1 und durchgeführt in einem einzelnen geschlossenen Rohrformat.

3. Verfahren nach Anspruch 1 oder 2 und wobei die Amplifikationsreaktion PCR, isotherme Amplifikation, RT-PCR umfasst.

4. Verfahren nach einem der Ansprüche 1 bis 3 und wobei eine nachfolgende Verarbeitung ein spektrales Entfalten der mehreren beobachteten Spektren umfasst, die sich aus den mehreren Signalen ergeben, die nachgewiesen werden.

5. Verfahren nach einem der vorhergehenden Ansprüche und wobei die Anregung durch einen Laser oder eine Leuchtdiode (*light emitting diode* - LED) abgegeben wird.

6. Verfahren nach einem der vorhergehenden Ansprüche und das ein Ausführen und ein Steuern der Reaktion unter Nutzen einer Peltierzelle umfasst, die eine Arbeitsfläche, die angeordnet ist, um die Reaktion zu erhitzen und zu kühlen, und eine Basisfläche aufweist, die bei einer konstanten Temperatur zwischen den oberen und den unteren Temperaturen der Reaktion gehalten wird.

7. Verfahren nach einem der vorhergehenden Ansprüche, wobei die emittierten Fluoreszenzsignale unter der Verwendung eines Spektrophotometers nachgewiesen werden, das optional kalibriert ist, um Licht bei 650 nm zu sammeln, das bis zu 750 nm reicht.

8. Verwendung einer Vorrichtung für den multiplexierten Nachweis von Nukleinsäurezielen gemäß dem Verfahren nach einem der Ansprüche 1-7, wobei die Vorrichtung durch eine Reaktionskammer (17), die zum Ausführen der Amplifikation angeordnet ist, und Mittel (18) für die Abgabe an die Probe aus rotem Anregungslicht, bei einer Wellenlänge von über 620 nm, und Aufweisen von Anregungsmittel (18) gekennzeichnet ist, die angeordnet sind, um mit einer Leistung von wenigstens 2 mW zu arbeiten.

9. Verwendung nach Anspruch 8, wobei die Vorrichtung ein Fluoreszenzsammelmittel aufweist, das ein Spektrophotometer (23) umfasst.

10. Verwendung nach einem der Ansprüche 8 oder 9, wobei die Vorrichtung angeordnet ist, um die Anregung in einem Bereich von 633-640 nm abzugeben.

11. Verwendung nach einem der Ansprüche 8-10, wobei die Vorrichtung Anregungsmittel (18) umfasst, die eine Laserdiode und eine Leuchtdiode (LED) umfassen.

12. Verwendung nach einem der Ansprüche 8-11, wobei die Vorrichtung eine Peltierzelle umfasst, die eine Arbeitsfläche, die angeordnet ist, um die Reaktion zu erhitzen und zu kühlen, und eine Basisfläche aufweist, die angeordnet ist, um bei einer konstanten Temperatur zwischen den oberen und den unteren Temperaturen der Reaktion gehalten zu werden.

13. Verwendung nach einem der Ansprüche 8-12, wobei die Vorrichtung eine Faseranordnung (13) umfasst, die angeordnet ist, um Licht zwischen der Lichtquelle (18) und dem Reaktionsgefäß und zwischen dem Reaktionsgefäß und einem Lichtdetektor zu übermitteln.

14. Verwendung nach einem der Ansprüche 8-13, wobei die Vorrichtung eine Anordnung von zwei mal vier Sammelfasern (13) umfasst, die nach einem Durchlaufen eines 665 nm-Langpassfilters (24) auf ein Spektrophotometer (23) derart fokussiert werden, dass ausschließlich das beabsichtigte Emissionslicht auf dem Spektrophotometer (23) abgebildet wird.

## Revendications

1. Procédé de détection multiplexée de cibles d'acide nucléique directement amplifiées dans une réaction avec un échantillon lorsque l'échantillon contient du sang, le procédé étant **caractérisé par** l'excitation de la réaction à une longueur d'onde supérieure à 620 nm et dans lequel l'excitation est effectuée avec une puissance d'au moins 2mW.

2. Procédé selon la revendication 1 et mis en oeuvre dans un seul format de tube fermé.

3. Procédé selon la revendication 1 ou la revendication 2 et dans lequel la réaction d'amplification comprend la PCR, l'amplification isothermique, la RT-PCR.

4. Procédé selon l'une quelconque des revendications 1 à 3 et dans lequel le traitement ultérieur comprend la déconvolution spectrale des multiples spectres observés provenant de la pluralité de signaux détectés.

5. Procédé selon l'une quelconque des revendications précédentes et dans lequel l'excitation est administrée par laser ou par une diode électroluminescente (DEL).

6. Procédé selon l'une quelconque des revendications précédentes et comprenant la réalisation et la régulation de la réaction en employant une cellule Peltier ayant une face de travail agencée pour chauffer et refroidir la réaction et une face de base maintenue à une température constante entre les températures supérieure et inférieure de la réaction.

7. Procédé selon l'une quelconque des revendications précédentes, dans lequel les signaux de fluorescence émis sont détectés par l'utilisation d'un spectrophotomètre, éventuellement étalonné pour collecter de la lumière à 650 nm dans une plage allant jusqu'à 750 nm.

8. Utilisation d'un appareil pour la détection multiplexée de cibles d'acide nucléique selon le procédé de l'une quelconque des revendications 1 à 7, dans laquelle l'appareil est **caractérisé par** une chambre de réaction (17) agencée pour effectuer l'amplification et un moyen (18) d'administration de lumière d'excitation rouge à l'échantillon, à une longueur d'onde supérieure à 620 nm, et ayant un moyen d'excitation (18) conçu pour fonctionner avec une puissance d'au moins 2 mW.

9. Utilisation selon la revendication 8, dans laquelle l'appareil a un moyen de collecte de fluorescence comprenant un spectrophotomètre (23).

10. Utilisation selon l'une quelconque des revendications 8 ou 9, dans laquelle l'appareil est agencé pour administrer une excitation dans une plage de 633 à 640 nm.

11. Utilisation selon l'une quelconque des revendications 8 à 10, dans laquelle l'appareil comprend un moyen d'excitation (18) comprenant une diode parmi une diode laser et une diode électroluminescente (DEL).

12. Utilisation selon l'une quelconque des revendications 8 à 11, dans laquelle l'appareil comprend une cellule Peltier ayant une face de travail agencée pour chauffer et refroidir la réaction et une face de base agencée pour être maintenue à une température constante entre les températures supérieure et inférieure de la réaction.

13. Utilisation selon l'une quelconque des revendications 8 à 12, dans laquelle l'appareil comprend un réseau de fibres (13) agencé pour transporter de la lumière entre la source de lumière (18) et le récipient de réaction et entre le récipient de réaction et un détecteur de lumière.

14. Utilisation selon l'une quelconque des revendications 8 à 13, dans laquelle l'appareil comprend un réseau de fibres de collecte de deux pouces par quatre (13) qui sont focalisées sur un spectrophotomètre (23) après avoir traversé un filtre passe-haut (24) de 665 nm de sorte que seul de la lumière d'émission prévue est imagée sur le spectrophotomètre (23).
